# EUROPEAN PATENT APPLICATION

(11) **EP 2 374 423 A1**
(43) Date of publication of application: **12.10.2011**
(21) Application number: 11250456.8
(22) Date of filing: 11.04.2011
(51) Int. Cl.: A61B 17/34

(54) **Universal height access port**

(30) Priority: 12.04.2010 US 323013 P; 21.02.2011 US 31346
(71) Applicant: Tyco Healthcare Group LP, New Haven, CT 06511 (US)
(72) Inventor: Kleyman, Gennady, Brooklyn, NY 11230 (US)
(74) Representative: Soames, Candida Jane

(57) **Abstract**

A surgical apparatus for positioning within a tissue tract accessing an underlying body cavity is adapted to tissues having different thicknesses. The surgical apparatus is configured to have different lengths. In one embodiment, the surgical includes a seal anchor member having two ends, one of which can be configured to fold and result in a plurality of folded states. Each folded state corresponds to a different length of the seal anchor member. The seal anchor member includes a slot to facilitate transition within the plurality of folded states. The seal anchor member further includes an aperture and a pin configured to further facilitate transition within the plurality of folded states.

## Description

### CROSS-REFERENCE TO RELATED APPLICATIONS

This application claims priority to, and the benefit of, U.S. Provisional Patent Application Serial Number 61/323,013, filed on April 12, 2010, the entire contents of which is hereby incorporated by reference.

### BACKGROUND

### Technical Field

The present disclosure relates generally to surgical apparatuses for use in minimally invasive surgical procedures, such as endoscopic and/or laparoscopic procedures, and more particularly, relates to a surgical apparatus that allows multiple surgical instruments to be inserted through a single incision.

### Description of Related Art

Today, many surgical procedures are performed through small incisions in the skin, as compared to the larger incisions typically required in traditional procedures, in an effort to reduce both trauma to the patient and recovery time. Generally, such procedures are referred to as "endoscopic", unless performed on the patient's abdomen, in which case the procedure is referred to as "laparoscopic." Throughout the present disclosure, the term "minimally invasive" should be understood to encompass both endoscopic and laparoscopic procedures.

During a typical minimally invasive procedure, surgical objects, such as surgical access devices (e.g., trocar and cannula assemblies) or endoscopes, are inserted into the patient's body through the incision in tissue. In general, prior to the introduction of the surgical object into the patient's body, insufflation gas is used to enlarge the area surrounding the target surgical site to create a larger, more accessible work area. Accordingly, the maintenance of a substantially fluid-tight seal is desirable so as to inhibit the escape of the insufflation gas and the deflation or collapse of the enlarged surgical site.

Different patients have different tissue thicknesses. Generally, access devices of different lengths are supplied in order to meet the various demands of patients based on their various tissue thicknesses. Such prior access devices as have been heretofore provided have had numerous disadvantages both from the standpoint of design as well as from the standpoint of availability of use.

From the design perspective, no one single prior access device is universally suitable for tissues having different thicknesses. In the prior art, each access device is designed in contemplation of a tissue having a particular thickness. Thus, access devices of different lengths have to be designed and supplied in order to accommodate patients with different needs based on their tissue thicknesses.

In the use of prior access devices, patient's tissue thickness needs to be assessed before performing a minimally invasive procedure. After assessing the tissue thickness and before performing the procedure, an access device having a length suitable for the patient's tissue thickness is selected. An error made in the assessment may lead to consequences adversely impacting the procedure. For instance, if the assessment underestimates the patent's tissue thickness, then an access device having a length less than the patient's tissue thickness may be selected. As a result, the selected access device is inadequate for the procedure, thus delaying the performance of the procedure.

Thus, to avoid the need of designing and supplying access devices of different lengths, to preclude the need of assessing tissue thickness, and to avoid unnecessary problems caused by the assessment, it is desirable to have a single access device that can be configured to different lengths, such that the single access device can be suitable for tissues having different thicknesses.

### SUMMARY

Disclosed herein is a surgical apparatus for positioning within a tissue tract accessing an underlying body cavity. The surgical apparatus comprises a seal anchor member. The seal anchor member has a longitudinal axis, a length, a first end and a second end. The first end is configured to fold along the longitudinal axis, resulting in a plurality of states. Each state corresponds to a different length of the seal anchor member.

In one embodiment, the plurality of states comprises a plurality of folded states. In each folded state, the first end has an outer surface and an inner surface. Each folded state is maintained by connecting the outer surface and the inner surface of the first end together. The plurality of folded states includes a maximum folded state and a minimum folded state. In the maximum folded state, the length of the seal anchor member is minimized. Likewise, in the minimum folded state, the length of the seal anchor member is maximized.

In another embodiment, the plurality of states further comprises an unfolded state in which the first end of the seal anchor member is not folded. Similar to the embodiment described above, the length of the seal anchor is minimized in the maximum folded state. Unlike the embodiment described above, the length of the seal anchor member is maximized in the unfolded state.

In a certain embodiment, the first end of the seal anchor member defines a slot to facilitate transition among the plurality of folded states. Further, the first end defines an aperture through which a pin is used to further facilitate transition between the maximum and minimum folded states. The pin further connects the outer surface and the inner surface together to maintain a folded state.

In another embodiment, a surgeon manually adjusts the length of the outer surface and the length of the inner surface of the first end in order to select a desired folded state. After making the selection, the surgeon uses a suture to hold the inner surface and the outer surface of the first end together for purposes of maintaining the selected folded state.

In a certain embodiment, the first end defines a substantially large radial diameter thereby increasing the range of motion of the surgical instruments inserted through the seal anchor member. The second end defines a substantially small radial diameter thereby providing easy insertion and removal of the seal anchor member through the tissues.

In a further embodiment, the length of the seal anchor member is substantially large for accommodating thick tissues in bariatric related procedures.

In another embodiment, the seal anchor member defines a coring configuration such that there is a large free open space within the seal anchor member for providing a large range of motion of the surgical instruments inserted therethrough.

In a preferred embodiment, the seal anchor member defines at least four longitudinal ports extending therethrough for accommodating surgical instruments.

### DESCRIPTION OF THE DRAWINGS

The above and other aspects, features, and advantages of the present disclosure will become more apparent in light of the following detailed description when taken in conjunction with the accompanying drawings in which:

**FIG. 1** is a front perspective view of a surgical apparatus in accordance with the principles of the present disclosure illustrating a seal anchor member positioned relative to the tissue;

**FIG. 2a** is a front perspective view of the seal anchor member of **FIG. 1** in the maximum folded state;

**FIG. 2b** is a front perspective view of the seal anchor member of **FIG. 1** in an intermediate folded state between the maximum and minimum folded states;

**FIG. 2c** is a front perspective view of the seal anchor member of **FIG. 1** in the minimum folded state;

**FIG. 3** is a front perspective view of the seal anchor member of **FIG. 1** illustrating the trailing end of the seal anchor member;

**FIG. 4** is a perspective view of the seal anchor member of **FIG. 1** illustrating the slot and the aperture of the trialing end;

**FIG. 5** is a top perspective view of the seal anchor member of **FIG. 1** illustrating a plurality of ports extending longitudinally therethrough;

**FIG. 6** is a side cross-sectional view of the seal anchor member of **FIG. 1** illustrating a port that extends longitudinally through the leading end and the intermediate portion of the seal anchor member;

**FIG. 7** is a front prospective view of an alternate embodiment of the seal anchor member shown in an unfolded state;

**FIG. 8** is a top prospective view of the seal anchor member of **FIG. 7****;**

**FIG. 9** is a front perspective view of the seal anchor member of **FIG. 7** shown in an unfolded state and shown disposed within a tissue tract having first thickness;

**FIG. 10** is a partially cutaway front perspective view of the seal anchor member of **FIG. 7** shown in an unfolded state and shown disposed within a tissue tract having a second thickness;

**FIG. 11** is a partially cutaway front perspective view of the seal anchor member of **FIG. 7** shown in a folded state and shown disposed within the tissue tract of **FIG. 10****;**

**FIG. 12** is a perspective view of the seal anchor member of **FIG. 7** shown in an unfolded state and shown disposed within the tissue tract of **FIG. 10****;**

**FIG. 13** is a perspective view of the seal anchor member of **FIG. 7** illustrated in a folded state and shown disposed within the tissue tract of **FIG. 10****;**

**FIG. 14** is a front prospective view yet another embodiment of a seal anchor member shown positioned relative to the tissue;

**FIG. 15** is a top perspective view of the seal anchor member of **FIG. 14****;**

**FIG. 16** is a front prospective view of an a still further embodiment of the seal anchor member; and

**FIG. 17** is a top perspective view of the seal anchor member of **FIG. 16****.**

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is farther from the user. In the following description, well-known functions or constructions are not described in detail to avoid obscuring the present disclosure in unnecessary detail.

One type of minimal invasive surgery described herein employs a device that facilitates multiple instrument access through a single incision. This is a minimally invasive surgical procedure, which permits a user to operate through a single entry point, typically the patient's navel. The disclosed procedure involves insufflating the body cavity and positioning a portal member within, e.g., the navel of the patient. Instruments including an endoscope and additional instruments such as graspers, staplers, forceps or the like may be introduced within the portal member to carry out the surgical procedure. An example of such a surgical portal is disclosed in commonly assigned U.S. patent application Serial No. 12/244,024, Pub. No. US 2009/0093752 A1, filed October 2, 2008, the entire content of which is hereby incorporated by reference herein.

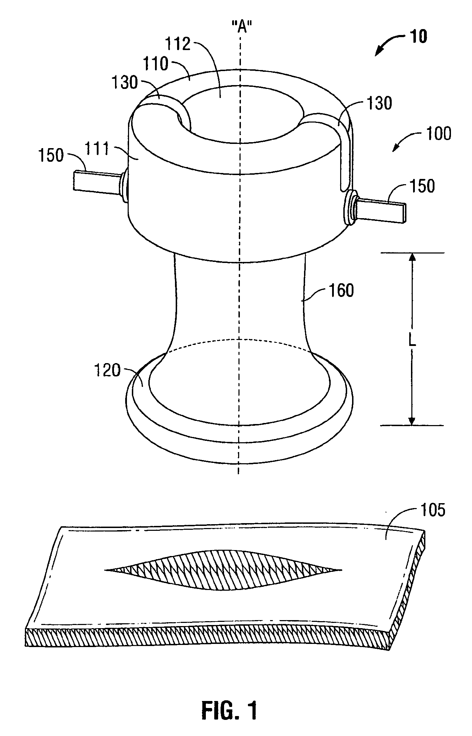

Referring now to the drawings, in which like reference numerals identify identical or substantially similar parts throughout the several views, **FIG. 1** illustrates a surgical apparatus **10** comprising a seal anchor member **100** in accordance with the principles of the present disclosure. Seal anchor member **100** is adapted for insertion within a tissue tract **105,** e.g., through the abdominal or peritoneal lining in connection with a laparoscopic surgical procedure. Seal anchor member **100** will be described in greater detail hereinbelow

With reference to **FIG. 1****,** seal anchor member **100** defines a longitudinal axis **"A"** and a length **"L"**. The length "L" describes the distance of the portion of the seal anchor member **100** that can be inserted through the tissue tract **105.** Seal anchor member **100** has respective trailing and leading ends **110, 120** and an intermediate portion **160** disposed between the trailing and leading ends **110, 120.** Seal anchor member **100** may be made from a semi-resilient, disposable, compressible, and flexible type material, for example, but not limited to, a suitable foam, gel material, or soft rubber having sufficient compliance to form a seal about one or more surgical objects, and also establish a sealing relation with tissue and with the surgical object. In one embodiment, the foam includes a polyisoprene material. As shown in **FIG. 1****,** seal anchor member **100** may define a substantially hourglass shape. However, it is contemplated that the seal anchor member **100** may define other configurations both prior and subsequent to insertion within tissue.

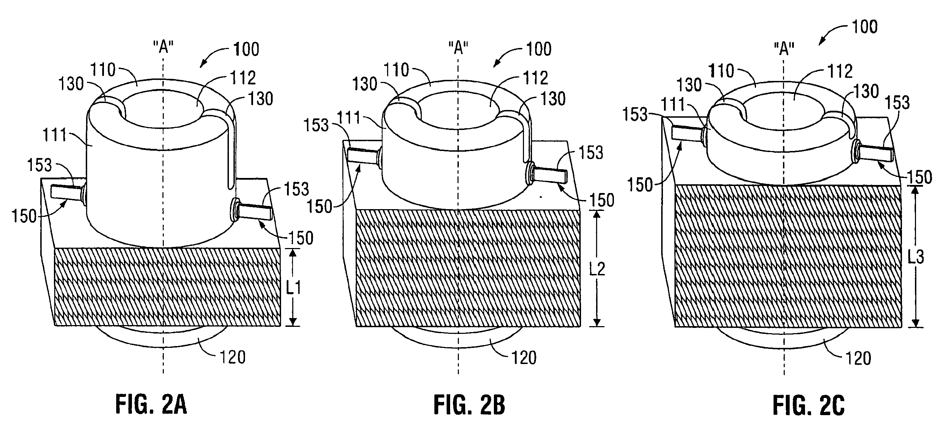

Due to the flexible and semi-resilient characteristics of seal anchor member **100,** the length "L" of seal anchor member **100** can be adjusted to be suitable for tissues of different thicknesses. In one embodiment, as illustrated in **FIG. 1****,** to configure seal anchor member **100** to have different lengths, trailing end **110** is configured to fold at any position along the longitudinal axis thus resulting in various seal anchor member lengths. As best illustrated in **FIGS. 2a - 2c****,** trailing end **110** is configured to define a plurality of folded states. Each of the three folded states in **FIGS. 2a - 2c** corresponds to a different length of seal anchor member **100:** "L1", "L2" and "L3". Trailing end **110** in each folded state defines an outer surface **111** and an inner surface **112.** The outer surface **111** is formed by the portion of the trailing end **110** that is folded along the longitudinal axis **"A"**, whereas the inner surface **112** is formed by the portion of the trailing end **110** that is not yet folded. As the trailing end **110** transits among different folded states, portions of the outer surface **111** may gradually merge into the inner surface **112,** and vice versa.

The plurality of the folded states range between a maximum folded state as shown in **FIG. 2a** and a minimum folded state as shown in **FIG. 2c****.** The maximum folded state describes a state in which a maximum portion of the trailing end **110** is folded. Accordingly, the length "L" of seal anchor member **100** is minimized, thus resulting in a minimum length "L" of seal anchor member **100.** In contrast, the minimum folded state happens when a minimum portion of the trailing end **110** is folded, resulting in a maximum length "L" of seal anchor member **100.** Thus, seal anchor member **100** can be configured to different lengths, and it can be adapted to different tissue thicknesses.

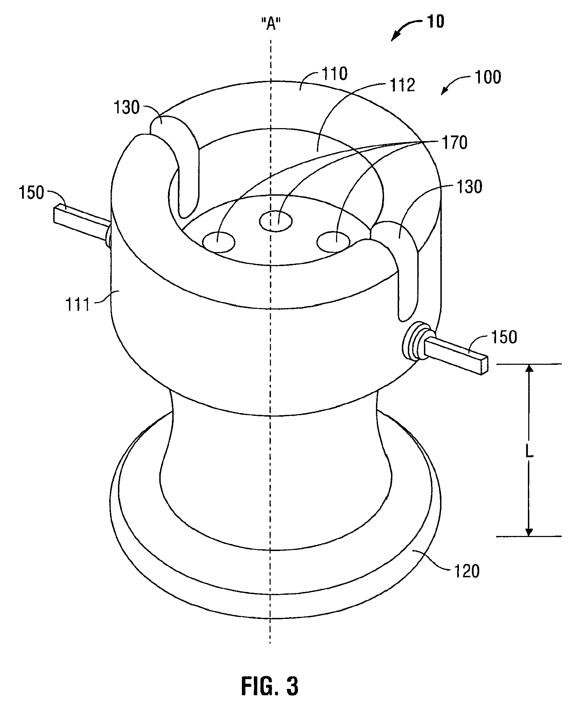

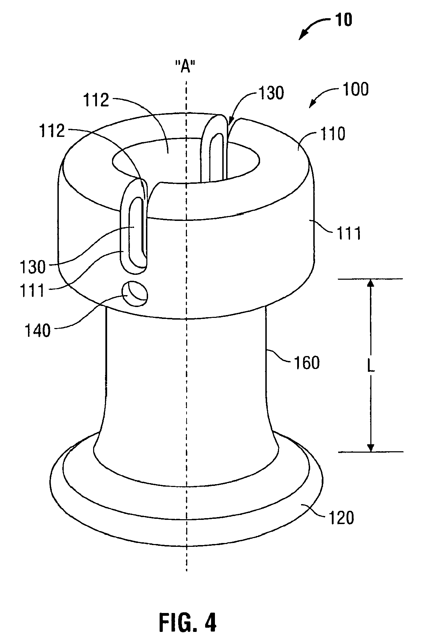

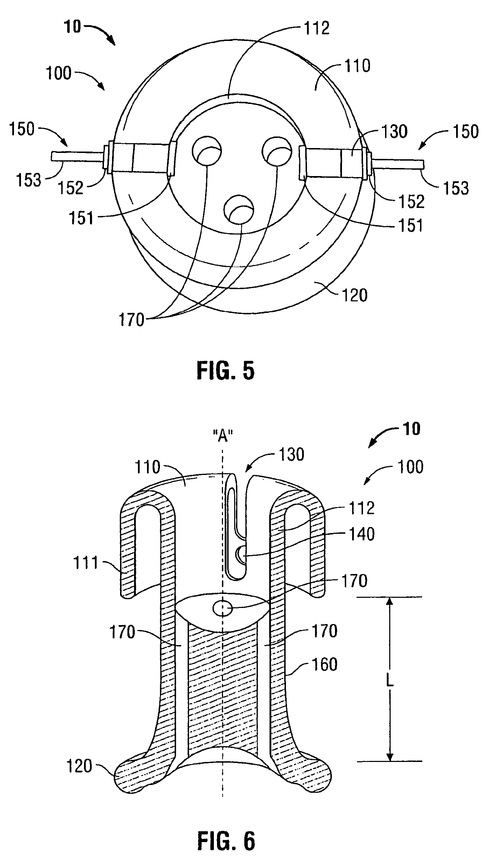

To facilitate transition between the maximum and minimum folded state, the trailing end **110** defines two slots **130,** as illustrated in **FIG. 3****.** Each slot **130** spans across the outer surface **111** and the inner surface **112** of the trialing end **110.** The two slots **130** are diametrically opposed on the trailing end **110.** As illustrated in **FIG. 4****,** each slot **130** corresponds to an aperture **140** which is defined by the trailing end **110.** Each aperture **140** is positioned directly beneath its corresponding slot **130** on the outer surface **111** of the trailing end **110.** With reference to **FIG. 5****,** each slot **130** further corresponds to a pin **150** which is disposed through the outer surface **111** and the inner surface **112** of the trailing end **110.** Each pin **150** includes a head **151,** a body **152,** and a handle **153.** The pin **150** is disposed through the outer surface **111** by having its body **152** positioned through the aperture **140** which is defined beneath the pin's **150** corresponding slot **130.** The body **152** is attached firmly to the outer surface **111** of the trailing end **110.** Further, the pin **150** protrudes beyond the outer surface and forms the handle **153** which can be used by a surgeon to adjust the position of pin **150** along the longitudinal axis "A." Further, the pin **150** extends through its corresponding slot **130** defined on the inner surface **112.** The portion of the pin **150** that extends beyond the inner surface **112** forms the head **151** of pin **150.** The head **151** has a diameter greater than that of the body **152.** The head **151** is placed against the inner surface **112** of the trialing end **110.**

With continued reference to **FIG. 5****,** the pin **150** is configured to slide through the slot **130** by lifting the handle **153** up and down along the longitudinal axis "A." Essentially, the slot **130** provides a passageway that guides the pin **150** to slide longitudinally under the control of the handle **153.** As the pin **150** slides from one end of the slot **130** to the other end of the slot **130,** the trailing end **110** undergoes a transition between the maximum folded state and the minimum folded state. Accordingly, the seal anchor member **100** transits between the minimum length "L" and the maximum length "L". Further details regarding this transition are explained below.

With reference to **FIGS. 2a-2c****,** as the handle **153** is lifted in an upward direction along the longitudinal axis "A", portions of the outer surface **111** gradually merge into the inner surface **112,** thus, transiting the trailing end **111** from a more folded state, as shown in **FIG. 2a****,** to a less folded state, as shown in **FIG. 2c****,** thereby increasing the length "L" of the seal anchor member **100.** In contrast, as the handle **153** is pushed in a downward direction along the longitudinal axis "A", portions of the inner surface **112** gradually merge into the outer surface **111,** thus, transiting the trailing end from a less folded state, shown in **FIG. 2c****,** to a more folded state, as shown in **FIG. 2a****,** thereby decreasing the length "L" of the seal anchor member **100.** Thus, the user can adjust the length "L" of the seal anchor member **100** by adjusting the handle **153** along the longitudinal axis "A" until a desired length is reached.

With reference to **FIGS. 5** and **6****,** the trailing end **110** exhibits an elongated, tubular structure defining a hollow center region, as shown in **FIG. 6****.** Seal anchor member **100** further includes at least on longitudinal port **170** extending along the longitudinal axis "A" of seal anchor member **100** between the leading end **120** and the intermediate portion **160.** The ports **170** are configured symmetrically with respect to the longitudinal axis "A". The ports **170** are spaced equidistant from the longitudinal axis "A". Each port 170 may be spaced equidistant from its neighboring ports. The longitudinal ports **170** are dimensioned to receive a surgical object, e.g. a surgical instrument (not shown) therethrough. Upon introduction of an instrument or surgical object (not shown) through a respective port **170,** the inner surface portions defining the port **170** establish and maintain a substantial sealed relation about the instrument or surgical object.

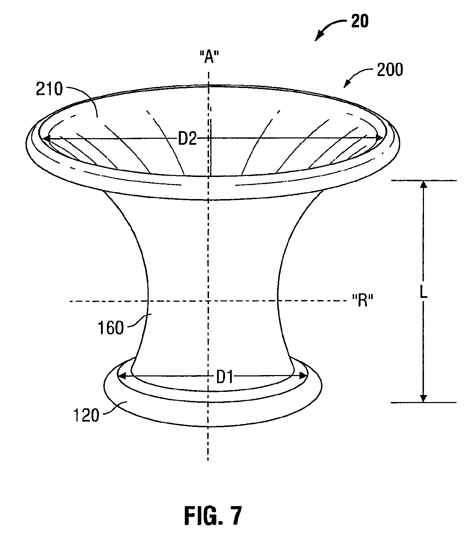

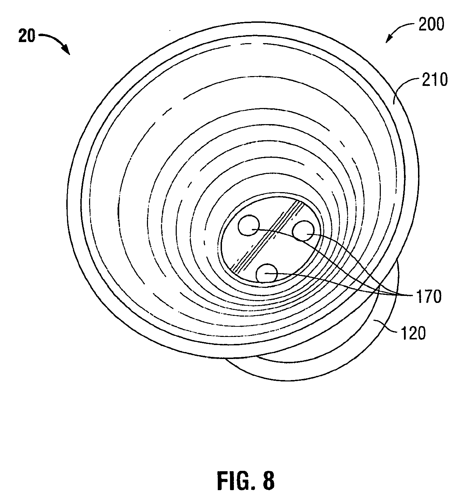

Turning now to FIGS. 7-13, a surgical apparatus **20** including a seal anchor member **200** will now be described. Seal anchor member **200** includes a leading end **120,** an intermediate portion **160,** and a trailing end **210.** A plurality of ports **170** is disposed between the intermediate portion **160** and the leading end **120** as illustrated in **FIG. 8****.** As illustrated in **FIG. 7****,** the trailing end **210** has an unfolded state in which the trailing end **210** is fully unfurled. The seal anchor member **200** reaches its maximum length "L" when the trailing end **210** is in the unfolded state. The leading end **120** defines a radial diameter **"D1"** relatively smaller than a radial diameter **"D2"** defined by the trailing end **210.** This particular configuration of the seal anchor member **200** with one end substantially small and the other end substantially large provides many benefits to surgical procedures. The leading end **120** with its relatively small dimension provides easy insertion and removal of the seal anchor member **200** through skin tissues, thus, reducing the time required to place and/or displace the seal anchor member **200** through the incisions during surgical operations and reducing tissue trauma. The trailing end **210** with its relatively large dimension creates a wide open space above the plurality of ports **170.** As a result, the trailing end **210** provides the surgeon a large free space to manipulate portions of the surgical instruments positioned above the ports 170, ultimately resulting in a significantly increased range of motion of the surgical instruments inserted through the seal anchor member **200** and facilitating off-axis motions of the surgical instruments. The relatively large trailing end **210** also facilitates transition among the plurality of the folded states as well as the unfolded state.

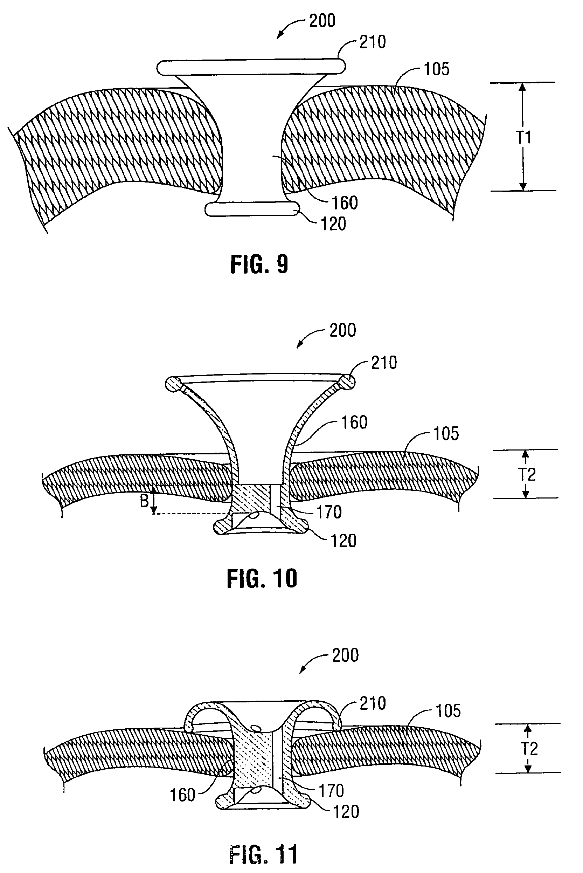

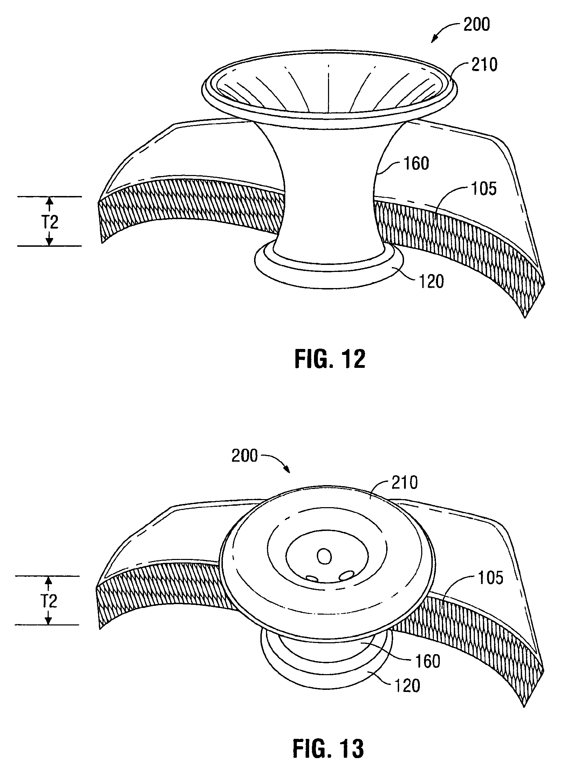

As shown in **FIGS. 9-13****,** adjustment of the length of the seal anchor member **200** to accommodate tissue tracts **105** having different thicknesses. As seen in **FIG. 9****,** tissue tract **105** has a thickness **T1** that corresponds to the overall length of seal anchor member **200** in an unfolded state. In such a situation, where the thickness of the tissue tract **105** corresponds to the unfolded length of the seal anchor member **200,** the seal anchor member **200** need not be folded. However, in situations in which the tissue tract **105** has a thickness, e.g., thickness **T2 (****FIGS. 10-13****),** that is less than the unfolded length of the seal anchor member 200, adjustment of the overall length of the seal anchor member **200** may be made to approximate thickness **T2,** as is illustrated in **FIGS. 11** and **13****.** As illustrated in **FIGS. 11** and **13****,** the trailing end **210** is folded such that the trailing end **210** is moved towards the surface of the tissue tract **105,** thereby reducing the distance between trailing and leading ends **210, 120** and substantially approximating thickness **T2.**

In a preferred embodiment, the surgical apparatus exhibits a coring configuration as illustrated in **FIG. 10****,** wherein the trailing end **210** and the intermediate portion **160** define a large free, open space in the center region of the seal anchor member **200** as if a large amount of materials has been removed therefrom. The intermediate portion **160** is filled with only a small amount of materials within the region **"B"** adjacent to the leading end **120,** as indicated in **FIG. 10****,** for defining the port **170.** Therefore, the port **170** in this particular embodiment is relatively small along the longitudinal axis **"A."** The coring configuration increases the flexibility of the seal anchor member **200** such that the overall shape of the seal anchor member **200** can be easily manipulated. For instance, due to the large empty space within the seal anchor member **200,** the seal anchor member **200** can be easily squeezed to facilitate its insertion through incisions. Since the seal anchor member **200** can be easily reduced to a small dimension due to squeezing, the incision through which the seal anchor member **200** to be placed can be created to have a relatively small opening thereby reducing the trauma experienced by the patients and subsequently reducing the patients' recovery time required to heal the incision. Further, the large free space defined above the port **170** increases the maneuverability and the range of motion of the surgical instruments inserted through the seal anchor member **200** and also significantly facilitates off-axis motions of the surgical instruments.

In a certain embodiment, the seal anchor member defines a substantially large length, such that the intermediate portion of the surgical apparatus is substantially lengthy along the longitudinal axis **"A"** of the seal anchor member between the trailing and leading ends. The seal anchor member further includes at least one longitudinal port substantially lengthy along the longitudinal axis "A" thereof between the intermediate portion and the leading end or between the trailing end and the leading end. The surgical apparatus of this embodiment is particularly designed to accommodate unusually thick tissues for purposes to be used in bariatric related treatment procedures. Obese patients have significantly thick tissues compared to patients of normal weight. During bariatric related treatment procedures, an incision is initially created off the midline for providing an access to the patient's body cavity. Access devices taught by the prior art are oftentimes not tall enough to be placed across the entire abdominal walls of obese patients. Thus, the prior access device cannot be securely placed within incisions, thereby adversely influencing the operation of bariatric procedures. The seal anchor member with a substantial length solves this problem. The seal anchor member can be securely placed at the incision extending across a very thick abdominal wall for introducing surgical instruments therethrough to manipulate tissues or organs within the body cavity. Thus, the seal anchor member is securely fit with respect to the incision, resulting in a stable state facilitating introduction of surgical instruments therethrough for performing bariatric procedures.

In a preferred embodiment, the seal anchor member defines at least four ports 170, with at least one port for accommodating an instrument connected to an insufflation or evacuation source.

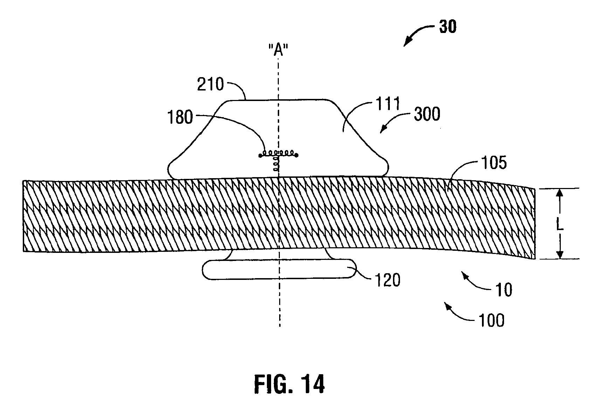

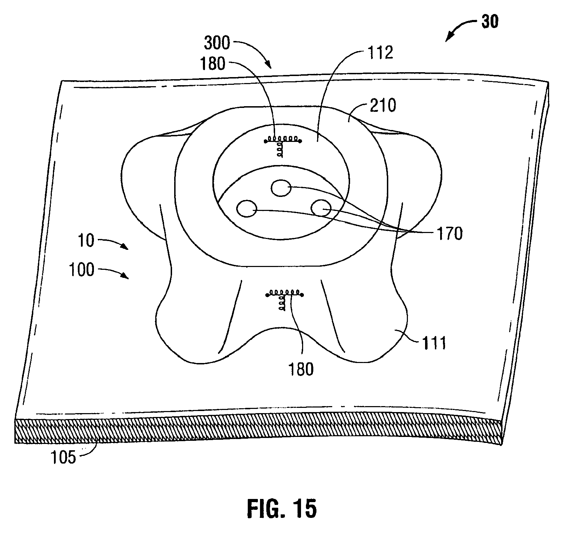

In a further embodiment, as illustrated in **FIGS. 14** **and** **15****,** a surgical apparatus **30** includes a seal anchor member **300** including a leading end **120,** a trailing end **210,** an inner surface **112,** an outer surface **111,** and a suture **180** adapted to maintain a selected folded state. Upon folding the trailing end **210** to achieve a desired folded state, suture **180** may be used to connect the inner surface **112** and the outer surface **111** of the trailing end **110** together for purposes of maintaining the selected folded state.

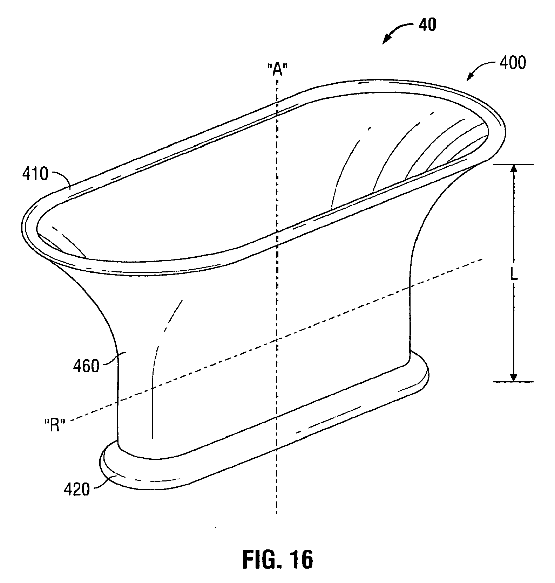

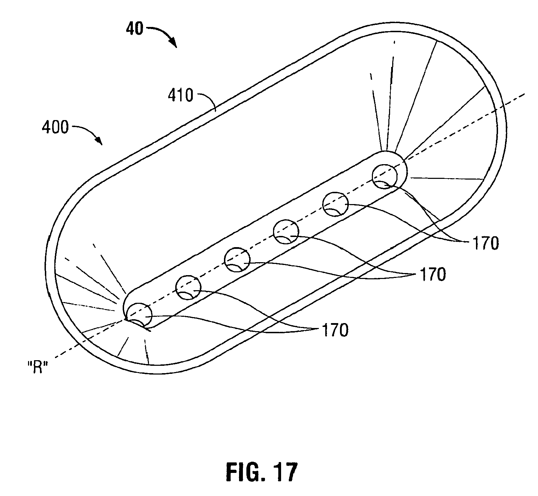

In a still further embodiment, as illustrated in **FIGS. 16** and **17****,** a surgical apparatus **40** includes a seal anchor member **400.** Seal anchor member **400** exhibits an elongated hourglass configuration and is substantially symmetrical with respect to radial axis **"R."** A plurality of ports **170** is disposed within the seal anchor member **400** and are arranged in a linear fashion along radial axis **"R,"** each port **170** including a lumen that is substantially parallel to longitudinal axis **"A"** of the seal anchor member **400.** The ports **170** may be equidistantly spaced apart.

Different embodiments of the disclosure may be combined with one another based on the particular needs of the patients to achieve optimal results of the surgical procedures. In one example, in bariatric related procedures, the seal anchor member may define a coring configuration having a substantial length for accommodating thick abdominal walls, and may comprise four longitudinal ports. In another example associated with bariatric related procedures, the seal anchor member may define a coring configuration having a substantial length for accommodating thick abdominal walls, and may further comprise a relatively small leading end, a relatively large trailing end and four longitudinal ports extending therethrough. Any of the presently disclosed embodiments may be used in procedures where access is achieved through a naturally occurring orifice (e.g. vagina or anus).

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragaphs:
1. A surgical apparatus for positioning within a tissue tract accessing an underlying body cavity, which comprises:
   a seal anchor member defining a longitudinal axis and a length, the seal anchor member including a first end and a second end, the first end configured to fold along the longitudinal axis, the first end defining a plurality of states, each state corresponding to a different length of the seal anchor member.
2. The surgical apparatus according to paragraph 1 wherein the plurality of states comprise a plurality of folded states.
3. The surgical apparatus according to paragraph 2 wherein the plurality of folded states include a maximum folded state such that the maximum folded state corresponds to a minimum length of the seal anchor member.
4. The surgical apparatus according to paragraph 2 wherein the plurality of folded states include a minimum folded state such that the minimum folded state corresponds to a maximum length of the seal anchor member.
5. The surgical apparatus according to paragraph 2 wherein each folded state has an outer surface and an inner surface of the first end.
6. The surgical apparatus according to paragraph 5 wherein each folded state is maintained by connecting the outer surface and the inner surface of the first end.
7. The surgical apparatus according to paragraph 2 wherein the first end defines a slot configured to facilitate transition within the plurality of folded states.
8. The surgical apparatus according to paragraph 2 wherein the first end defines an aperture through which a pin is configured to facilitate transition within the plurality of folded states.
9. The surgical apparatus according to paragraph 1 wherein the plurality of states comprise an unfolded state, wherein the unfolded state corresponds to a maximum length of the seal anchor member.
10. The surgical apparatus according to paragraph 1 further comprising an intermediate portion disposed between the first and second ends along the longitudinal axis.
11. The surgical apparatus according to paragraph 10 further comprising at least one longitudinal port extending between the intermediate portion and the second end and being configured for substantially sealed reception of an object therein.
12. The surgical apparatus according to paragraph 11 wherein the at least one longitudinal port is configured symmetrically with respect to the longitudinal axis.
13. The surgical apparatus according to paragraph 11 wherein the at least one longitudinal port is arranged in a linear fashion along a radial axis of the seal anchor member.
14. The surgical apparatus according to paragraph 1 wherein the trailing end exhibits a tubular configuration.
15. The surgical apparatus according to paragraph 1 wherein the seal anchor member exhibits an hourglass configuration.
16. The surgical apparatus according to paragraph 1 wherein the seal anchor member exhibits an hourglass configuration elongated in a radial axis of the seal anchor member.

## Claims

1. A surgical apparatus for positioning within a tissue tract accessing an underlying body cavity, which comprises:
a seal anchor member defining a longitudinal axis and a length, the seal anchor member including a first end and a second end, the first end configured to fold along the longitudinal axis, the first end defining a plurality of states, each state corresponding to a different length of the seal anchor member.

2. The surgical apparatus according to claim 1 wherein the plurality of states comprise a plurality of folded states.

3. The surgical apparatus according to claim 2 wherein the plurality of folded states include a maximum folded state such that the maximum folded state corresponds to a minimum length of the seal anchor member.

4. The surgical apparatus according to claim 2 wherein the plurality of folded states include a minimum folded state such that the minimum folded state corresponds to a maximum length of the seal anchor member.

5. The surgical apparatus according to any of claims 2 to 4 wherein each folded state has an outer surface and an inner surface of the first end.

6. The surgical apparatus according to claim 5 wherein each folded state is maintained by connecting the outer surface and the inner surface of the first end.

7. The surgical apparatus according to any preceding claim wherein the first end defines a slot configured to facilitate transition within the plurality of folded states.

8. The surgical apparatus according to any of claims 1 to 6 wherein the first end defines an aperture through which a pin is configured to facilitate transition within the plurality of folded states.

9. The surgical apparatus according to claim 1 wherein the plurality of states comprise an unfolded state, wherein the unfolded state corresponds to a maximum length of the seal anchor member.

10. The surgical apparatus according to any preceding claim further comprising an intermediate portion disposed between the first and second ends along the longitudinal axis.

11. The surgical apparatus according to claim 10 further comprising at least one longitudinal port extending between the intermediate portion and the second end and being configured for substantially sealed reception of an object therein.

12. The surgical apparatus according to claim 11 wherein the at least one longitudinal port is configured symmetrically with respect to the longitudinal axis.

13. The surgical apparatus according to claim 11 wherein the at least one longitudinal port is arranged in a linear fashion along a radial axis of the seal anchor member.

14. The surgical apparatus according to any preceding claim wherein the trailing end exhibits a tubular configuration.

15. The surgical apparatus according to any preceding claim wherein the seal anchor member exhibits an hourglass configuration.

16. The surgical apparatus according to claim 15 wherein the seal anchor member exhibits an hourglass configuration elongated in a radial axis of the seal anchor member.
